# EUROPEAN PATENT SPECIFICATION

(11) **EP 0 569 548 B1**
(45) Date of publication and mention of the grant of the patent: **29.10.1997**
(21) Application number: 92907087.8
(22) Date of filing: 28.01.1992
(51) Int. Cl.: A61B 17/20, A61B 17/39

(54) **CATHETER WITH ELECTROSURGICAL CUTTER**
KATHETER MIT ELEKTRO-CHIRURGISCHEM SCHNEIDINSTRUMENT
CATHETER A LAME ELECTROCHIRURGICALE

(30) Priority: 29.01.1991 US 647472; 31.01.1991 US 649034
(43) Date of publication of application: 18.11.1993
(73) Proprietor: APPLIED MEDICAL RESOURCES, INC., Laguna Hills, CA 92653 (US)
(72) Inventor: CLAYMAN, Ralph V., St. Louis, MO 63105 (US)
(74) Representative: Enskat, Michael Antony Frank
(86) International application number: US9200766
(87) International publication number: WO9212762

(56) References cited:
- WO-A-91/17714
- DE-A- 3 402 573
- SU-A- 938 977
- US-A- 4 273 128
- US-A- 4 484 579
- US-A- 4 793 348
- US-A- 4 799 479
- US-A- 4 919 133
- US-A- 5 053 044
- US-A- 5 080 660

## Description

The present invention relates to catheter assemblies.

In radiofrequency electrosurgical cutting a radiofrequency current is allowed to pass from an active cutting electrode through a patient's tissue and into a grounding pad or cable. The current cuts tissue at the active cutting electrode, the cutting rate being dependant on current density through the tissue in that area. With a low current density heat is generated but no cut is achieved. With a high current density fast cutting occurs.

Current density depends upon the voltage applied to the electrosurgical circuit and the series impedance or resistance to current flow of that circuit. Current density is also dependent upon the area the active cutting electrode presents to the patient's tissue. The smaller this area, the higher the current density. Since the area of the active electrode is fixed for a specific cutter, and the series impedance of the circuit is beyond the surgeon's control, the current density is typically adjusted by varying the voltage applied to the electrode. This adjustment is typically present on conventional electrosurgical generators.

The series impedance is dependent upon several factors which are outside the control of the surgeon. These factors may include the material and design of the active electrode, the type of tissue to be cut, and the location of the grounding pad relative to the cutting site. Generators used in this type of surgery have a wide range of power output to accommodate a variety of procedures and devices. For example, the generator can be adjusted to either cut tissue or to merely cauterize previously cut or torn tissue.

The objective in electrosurgical cutting is to heat the cells of the tissue so rapidly that they explode into steam leaving a cavity in the cell matrix. The heat is meant to be dissipated in the steam and not to dry out adjacent cells. When the electrode is moved and fresh tissue is contacted, new cells are exploded and the incision is made. The current utilized in electrosurgical cutting is in the radiofrequency range and operates by jumping across an air gap to the tissue. This is commonly referred to as sparking.

An explanation of electrosurgical cutting theory can be found in the FORCE 1 Instruction Manual published by Valleylab of Boulder, Colorado, on March 1, 1986.

An advantage of electrosurgical cutting, particularly if it is performed utilizing a cutting electrode as disclosed in PCT Publication No WO 91/17714, is that overheating of adjacent tissue with accompanying desiccation and damage is limited or prevented. Thus, this procedure provides a clean cut without damage to adjacent tissue. A clean controlled cut is particularly desirable to assure that tearing does not occur in a direction away from the desired orientation of the cut.

Dilation catheters are used to dilate body vessels, orifices and conduits such as an artery narrowed by atherosclerotic plaque or a urethra constructed by enlarged prostate. These catheters basically consist of an elongate cannula having an inflatable non-extensible balloon or bladder at or near its distal end. A guide wire or other axial support means is often included to improve the torque control or "steerability" of the catheter.

The major advantage of using a dilation catheter instead of conventional surgery is that it is less invasive. Nevertheless, the dilation processes of the past can also result in significant trauma. As the elastomeric bladder expands, it exerts pressure on the surrounding tissue, causing the tissue to compress, deform and expand. The tissue, of course, has an inherent limit of deformability. When the dilation pressure causes the tissue to deform beyond that limit, the tissue tears apart, often to form a jagged wound.

German Patent 3,402,573 is concerned with a single lumen multi-purpose catheter having an extensible elastic balloon with a cutting facility for treatment of stenosis. This patent utilizes three balloons of equal size at the distal end of the catheter. Each elastomeric balloon carries small cutter elements which extend in the longitudinal direction and which are held in a trough made of hard rubber or plastic. Prior to use the cutters lie hidden in longitudinal slots of the relatively thick wall of the one-lumen catheter. Threads anchor the plate when the balloons are inflated thereby limiting the degree of penetration of adjacent plaque and possibly tissue.

German Patent No DE-A-2,426,781 discloses a balloonless catheter which has a flexible tubular end portion. A wire electrode has an exposed portion which extends from the distal end to an opening spaced from the distal end. By withdrawing the wire into the tube, the flexible end flexes and the wire forms a chord across the now arcuate form of the end. The wire can now be energised by high frequency energy to sever any tissue that it comes into contact with.

The object of the present invention is to provide an improved balloonless catheter.

According to the present invention there is provided a balloonless catheter assembly comprising an elongate tubular member having a distal end portion insertable into a predetermined position in a body conduit, a cutting member supported by said distal end portion and having an external component which extends axially from an opening in the tubular member to a fixed location spaced from said opening characterised by finger tab means extending through slot means in the tubular member into engagement with the cutting means therein, and displaceable axially along the slot to flex said component of the cutting member radially of the distal end portion into and out of engagement with the body conduit to effect incision thereof.

A balloonless catheter will now be described, by way of example, with reference to the accompanying diagrammatic drawings, in which:
Figure 1 is a partly cross-sectional, isometric view of the dilation catheter of PCT Publication No. WO 91/17714 and which is not an embodiment of the present invention.
Figure 2 is a cross-sectional view taken along line 2-2 of Figure 1;
Figure 3 is an axial cross-section view of a balloonless catheter embodying the invention; and
Figure 4 is an axial cross-sectional view of a further embodiment of the invention wherein the cutting element is fixed to the catheter at a location proximal to the distal end of the catheter.

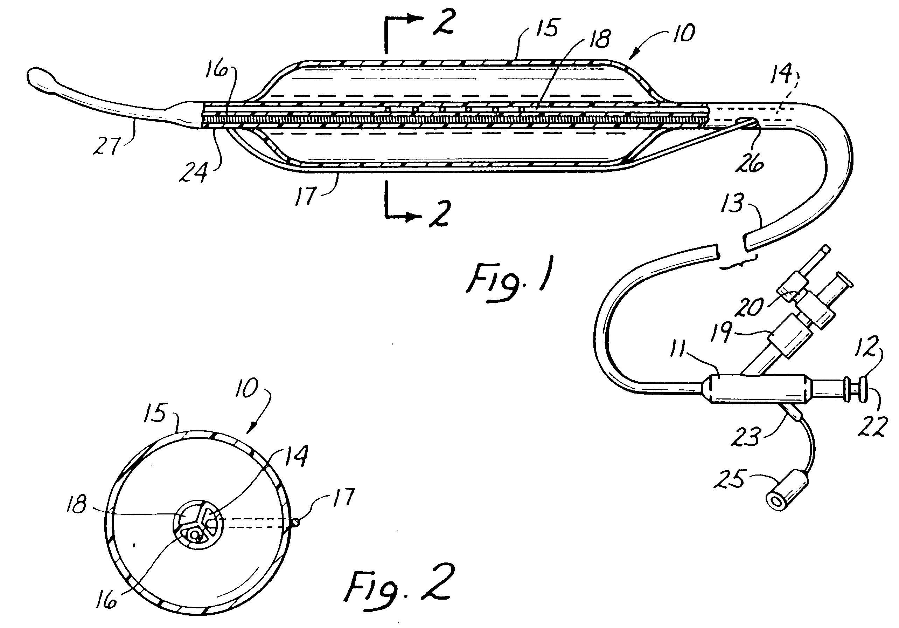

Figure 1 depicts a dilation catheter assembly, generally designated 10, that may be used for dilating a body vessel or conduit, such as a ureter or urethra, to treat a blockage or other obstruction. The main elements of the catheter assembly 10 are: an adapter 11 that defines the proximal end 12 of the assembly 10 and a site for various ports to the assembly 10; a catheter body 13 having a triple lumen 14 (Figure 2); an inflatable balloon or bladder member 15; a stiffening guide wire or stylet 16 that extends longitudinally within one of the three lumens 14 of the catheter body 13; and a cutting element or electrode 17, preferably a radiofrequency cutting element 17 activatable by a radiofrequency power source 21. The electrosurgical cutting element 17 is in the nature of a wire which extends generally parallel to the longitudinally extending inflatable bladder 15.

In use, the bladder 15 is inserted into a body conduit vessel or orifice to a location where a surgical cut is required. The bladder 15 is then inflated (an inextensible bladder is generally used) with radiofrequency current being passed through the cutting element 17. This leads to the wire being moved outwardly and incising adjacent tissue in that direction.

The material used for the wire can be any of the materials currently used for electrosurgical cutting. For example, the wire can be made of stainless steel or tungsten. As illustrated in Figure 2 herein, one of the three lumens 14 serves as an inflation/deflation passageway 18, a second lumen carries the guidewire or stylet 16 and serves as a drainage/infusion passageway, and a third lumen carries the cutting element 17. A sheath surrounding the cutting element 17 can be provided with a slit facing away from the bladder 15.

The inflatable balloon or bladder member 15 is preferably of the inextensible or constant volume variety, that is it can, when expanded, assume only a specific size and shape. Thus, the balloon member 15 cannot extend or bulge longitudinally within the body conduit beyond its predetermined diameter or length. Since a non-distensible balloon member 15 cannot extend longitudinally, as can elastic or elastomeric balloons, it must exert the force caused by inflation of the balloon member 15 radially against an enclosing body conduit or the like. In contrast, if an elastic or elastomeric balloon is expanded within a body conduit which has one portion particularly narrowed and particularly resistant to expansion, the balloon will simply elongate rather than acting radially outwardly against the constriction.

It is preferred to utilize a radiofrequency cutting element 17 for a number of reasons. One reason is that a radiofrequency cutting element 17 will not perform any cutting unless and until it is activated by passing a radiofrequency current through it. As a result, accidental cuts cannot be made away from the area where cutting is desired. And second, with proper control, cutting can be very sharply defined leading to a clean incision without tearing. This radiofrequency cutting or cauterizing technique can, thus, provide significant advantages over the use of prior art cutters in an apparatus of the nature disclosed herein.

The adaptor 11 serves as a site for a bladder inflation/deflation port 19 that is attached to a source of inflation medium (not shown) for inflating the bladder member 15, or to a suction source (not shown) for deflating the bladder member 15. Port 19 has a valve 20 for regulating the inflation medium or suction, as the case may be. Port 19 connects into the proximal end of an inflation/deflation passageway 18 that extends from the port 19 to the bladder member 15. The adaptor 11 also serves as a site for the drainage tube inlet/outlet port 22 and a cutting element port 23. The drainage port 22 is connected to the proximal end of the lumen that carries the guide wire or stylet 16. The drainage port 22 may serve as a site for removing fluid from the lumen or as a site for infusing fluid into the lumen.

The distal end of the catheter body has a series of drain holes 24 to facilitate flushing the lumen with fluid or voiding the bladder member 15. A "banana plug" cutting element connector 25 is affixed to the end of the cutting element port. The cutting element 17 extends from the connector 25 through the lumen of the catheter body 13, exits therefrom via an aperture 26, and continues along the exterior of the bladder member 15.

The cutting element 17 can consist of a thin wire which has an external incising edge that faces outwardly from the bladder member 15. Alternatively, the cutting element 17 may be a sharp edge, beam or, more preferable, a radiofrequency cutting or cauterizing element 17. The element 17 and bladder member 15 are constructed such that the cutting element 17 is carried on the exterior of the bladder member 15 (at least when the bladder member is inflated) but is not capable of incising the bladder member 15.

For use in urethral dilation the distal end of the assembly 10 includes a coudet tip 27. Such structure may not be necessary or desirable for dilating other conduit/orifices. For urethral dilation, the assembly 10 may optionally include another lumen and "Foley" type balloon (not shown) distally of the dilation bladder member 15 to anchor the catheter in the bladder neck of the human body and thereby facilitate correct positioning of the dilation bladder member 15. This has the further advantage of minimizing the possibility of migration and displacement of the assembly 10. One or more of the catheter assembly components may be made of radiopaque materials to facilitate the visualization of the assembly 10 by the physician during placement of the assembly 10 in the body vessel/conduit.

The typical surgical procedure in which the catheter assembly 10 is employed, involves the following steps. Normally a cytoscope is first inserted into the vessel/conduit/orifice to be dilated. Calibration devices may be inserted through the cytoscope to facilitate measuring the extent of the vessel/conduit/orifice being dilated. The dilation catheter of Figure 1 is then inserted to the desired depth in the vessel/conduit and positioned using fluoroscopic and/or X-ray techniques. A cytoscope lens may then be inserted through the catheter body 13 to allow visualization of the catheter and the bladder location. Fluid may be infused through the cytoscope to facilitate such visualization.

Once in position, the bladder member 15 is inflated. Such inflation causes the cutting element 17 to move radially outwardly as the bladder surface expands radially until the cutting element 17 contacts the surrounding tissue. As used herein the term "tissue" is intended to include, without limitation, normal tissue, neoplastic tissue (tumours) or an obstruction such as plaque. Preferably the bladder member 15 is non-distensible.

Continued radial expansion of the bladder member 15 positions the cutting element 17 and causes the bladder member 15 to exert pressure on the tissue thereby subjecting the tissue to a substantially uniform tangential tension. Then a radiofrequency current can be passed through the cutting element 17.

This combined cutting and dilating action results in the tissue being expanded without being torn due to a build-up of excess stresses within the tissue. Instead, the tissue is cut in a clean, concentrated, generally longitudinal fashion by the cutting element 17 and the dilation does not uncontrollably tear the tissue and cause excessive trauma and bleeding. The inflated bladder member 15 provides the additional benefit of acting as a tamponade to reduce bleeding.

After the vessel/conduit/orifce tissue is incised and dilated, and the blockage/obstruction is relieved, the power through the radiofrequency cutting element 17 is discontinued. Then the bladder member 15 can be deflated by operation of the inflation/deflation port valve 20. Deflation of the bladder member 15 permits a simultaneously radial retraction of the cutting element 17 out of contact with the tissue. As the bladder member 15 is deflated the cutting element 17 may be retracted via the connector 25. If desired, the cutting element 17 may be retracted prior to complete deflation of the bladder member 15 and/or the bladder member 15 reinflated and left in place to act as a tampon. Alternatively, the catheter can be withdrawn from the vessel/conduit altogether.

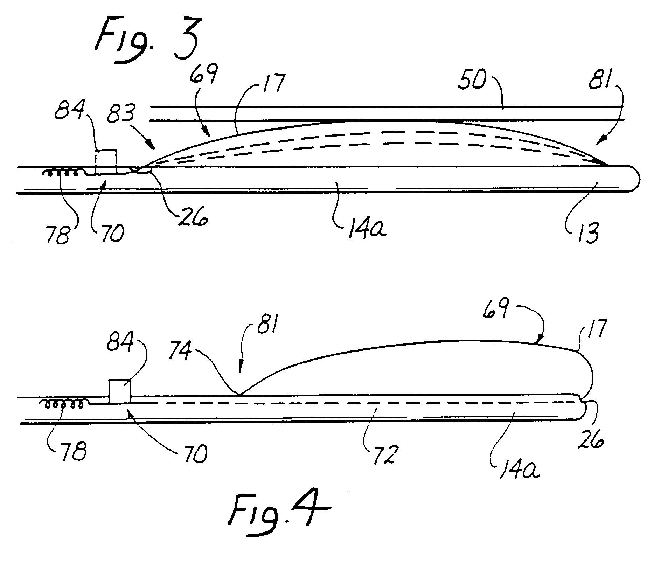

The catheter shown in Figures 3 and 4 differs from the previously disclosed catheter in that instead of a balloon the means for advancing the cutting element 17 into proximity with the tissue, comprises a finger tab 84. This tab 84 engages the cutting element 17 preferably through a slot in the body 13 of the catheter. As the tab 84 is moved distally of the catheter 10, the cutting element 17 passes through the aperture 26 to increase the length of the exterior portions 69 relative to the interior portions 70. By increasing the length of the exterior portions 69, the cutting element 17 tends to move outwardly, radially into proximity with the urethra 50.

As the tab 84 moves distally of the catheter 10, the exterior portions 69 of the cutting element 17 assume progressive positions having a particular configuration. In most cases, this particular configuration will at least be arcuate. In Figure 3 wherein the aperture 26 is disposed proximally of this exterior portion 69, the particular configuration tends to be parabolic. Thus each of the progressive positions of the exterior portions 69 define a parabola. In other embodiments, this particular configuration may be ovoid.

In Figure 4, wherein the aperture 26 is disposed distally of the exterior portions 69, the particular configuration tends to take the shape of one side of a heart. Each of these configurations may have a particular advantage in the context of a particular operative procedure.

After the catheter 10 has been positioned, and the cutting element 17 has been operationally deployed into contact with the tissue, the element 17 can be activated in the manner previously discussed. When the tissue has been cut thereby enlarging the circumferential dimension of the passage or cavity, the cutting element 17 can be withdrawn from proximity to the tissue into compliance with the catheter body 13. This is accomplished by moving the finger tab 84 proximally thereby drawing the cutting element into the lumen 14a of the catheter 10. By moving the finger tab 84 proximally of the catheter 10, the cutting element 17 is drawn through the aperture 26 thereby decreasing the length of the exterior portions 69 relative to the interior portions 70.

As will be appreciated, the cutting element 17 includes an interior portion 70 which is disposed in the lumen 14a and an exterior portion 69 which is disposed outside of the catheter body 13. The finger tab 84 provides means for increasing the length of the exterior portions 69 so that the cutting element 17 is moved radially outwardly into contact with the tissue. The cutting element 17 thus moves radially into proximity with the catheter body 13 when the distal force on the finger tab 84 is relieved. The spring 78 provides a proximal tension on the cutting element 17 decreasing the length of the exterior portions 69 while increasing the length of the interior portions 70.

## Claims

1. A balloonless catheter assembly comprising an elongate tubular member (13) having a distal end portion insertable into a predetermined position in a body conduit, a cutting member (17) supported by said distal end portion and having an external component (69) which extends axially from an opening (26) in the tubular member (13) to a fixed location spaced from said opening (26) characterised by finger tab means (84) extending through slot means in the tubular member (13) into engagement with the cutting means (17) therein, and displaceable axially along the slot to flex said component (69) of the cutting member (17) radially of the distal end portion into and out of engagement with the body conduit to effect incision thereof.

2. An assembly according to Claim 1, characterised by means (49) for electrically activating the cutter member (17) to cut the conduit.

3. An assembly according to Claim 1 or to Claim 2, characterised in that the cutting member (17) comprises a thin wire.

4. An assembly according to any preceding claim, characterised by an open ended sheath from which the distal end portion of the tubular body (13) may be ejected for use, and into which the tubular body (13) may be retracted after use.

5. A catheter assembly according to any preceding claim, characterised in that the cutting member (17) comprises a radiofrequency electrosurgical cutting element.

6. An assembly according to any preceding claim, characterised by means disposed between the cutting member (17) and the distal end portion of the tubular member (13) for directing energy from the cutting member (17) away from the distal end portion and towards the conduit.

7. An assembly according to any preceding claim, characterized in that the external component (69) of the cutting member (17) assumes progressive shapes in response to progressive movement of the finger tab means (84), each of the progressive shapes having a particular configuration.

8. An assembly according to Claim 7, characterised in that the particular configuration includes portions of an oval shape.

9. An assembly according to Claim 7, characterized in that the particular configuration includes portions of a parabolic shape.

10. An assembly according to Claim 7, characterized in that the particular configuration includes portions of a part of a heart shape.

## Patentansprüche

1. Ballonlose Katheteranordnung, enthaltend ein langgestrecktes, röhrenförmiges Glied (13) mit einem distalen Endabschnitt, der an eine vorbestimmte Stelle eines Körpergangs eingebracht werden kann, ein auf dem distalen Endabschnitt gelagertes Schneidglied (17) mit einem Außenabschnitt (69), der sich axial von einer Öffnung (26) in dem röhrenförmigen Glied (13) bis zu einem entfernt von der Öffnung (26) befindlichen Fixpunkt erstreckt, gekennzeichnet durch ein Fingerstellmittel (84), das sich durch einen Schlitz in dem röhrenförmigen Glied (13) erstreckt und in Eingriff mit dem darin befindlichen Schneidglied (17) ist und das längs des Schlitzes axial verschiebbar ist, um den Abschnitt (69) des Schneidgliedes (17) gegenüber dem distalen Endabschnitt in und außer Eingriff mit dem Körpergang radial zu biegen, um einen Schneidvorgang auszuführen.

2. Katheteranordnung nach Anspruch 1, gekennzeichnet durch Mittel (49) für die elektrische Aktivierung des Schneidglieds (17) zum Schneiden des Körpergangs.

3. Katheteranordnung nach Anspruch 1 oder 2, dadurch gekennzeichnet, dass das Schneidglied (17) mit einem dünnen Draht ausgerüstet ist.

4. Katheteranordnung nach einem der vorerwähnten Ansprüche, gekennzeichnet durch eine Hülle mit offenem Ende, von der aus der distale Endabschnitt des röhrenförmigen Glieds (13) in die Betriebsstellung ausgestoßen und in die das röhrenförmige Glied (13) nach dem Betrieb zurückgezogen werden kann.

5. Katheteranordnung nach einem der vorerwähnten Ansprüche, dadurch gekennzeichnet, dass das Schneidglied (17) aus einem Hochfrequenz-Elektrotomieaggregat besteht.

6. Katheteranordnung nach einem der vorerwähnten Ansprüche, gekennzeichnet durch Mittel, die zwischen dem Schneidglied (17) und dem distalen Endabschnitt des röhrenförmigen Glieds (13) angeordnet sind und die Energie von dem Schneidglied (17) von dem distalen Endabschnitt weg und auf den Körpergang hin leiten.

7. Katheteranordnung nach einem der vorerwähnten Ansprüche, dadurch gekennzeichnet, dass der Außenabschnitt (69) des Schneidglieds (17) infolge der schrittweisen Bewegung des Fingerstellmittels (84) schrittweise Formen annimmt, wobei jede der schrittweise eingenommen Formen eine besondere Konfiguration bildet.

8. Katheteranordnung nach Anspruch 7, dadurch gekennzeichnet, dass die besondere Konfiguration ovale Abschnitte aufweist.

9. Katheteranordnung nach Anspruch 7, dadurch gekennzeichnet, dass die besondere Konfiguration parabolische Abschnitte aufweist.

10. Katheteranordnung nach Anspruch 7, dadurch gekennzeichnet, dass die besondere Konfiguration Abschnitte aufweist, die einer geteilten Herzform entsprechen.

## Revendications

1. Ensemble de cathéter sans ballon, comprenant un élément tubulaire allongé ( 13 ) ayant une portion d'extrémité distale insérable, dans une position prédéterminée, dans un conduit de corps, un élément coupant ( 17 ) supporté par ladite portion d'extrémité distale et ayant une partie extérieure ( 69 ) qui s'étend axialement depuis une ouverture ( 26 ), dans l'élément tubulaire ( 13 ) à un emplacement fixe espacé de ladite ouverture ( 26 ), caractérisé par un moyen formant curseur (84) qui s'étend dans un moyen de fenêtre formé dans l'élément tubulaire ( 13 ) pour venir s'engager avec le moyen coupant ( 17 ) placé à l'intérieur, et qui peut se déplacer axialement le long de la fenêtre pour infléchir ladite partie ( 69 ) de l'élément coupant ( 17 ) radialement par rapport à la portion d'extrémité distale pour s'engager avec le conduit de corps, ou se dégager de celui-ci, afin de l'inciser.

2. Ensemble selon la revendication 1, caractérisé par un moyen ( 49 ) pour exciter électriquement l'élément coupant ( 17 ) afin de couper le conduit.

3. Ensemble selon la revendication 1 ou 2, caractérisé en ce que l'élément coupant ( 17 ) comporte un fil fin.

4. Ensemble selon l'une quelconque des revendications précédentes, caractérisé par une gaine, ouverte à son extrémité, de laquelle la portion d'extrémité distale du corps tubulaire ( 13 ) peut être sortie pour utilisation, et dans laquelle le corps tubulaire ( 13 ) peut être rentré après utilisation.

5. Ensemble de cathéter selon l'une quelconque des revendications précédentes, caractérisé en ce que l'élément coupant ( 17 ) comprend un élément coupant électrochirurgical utilisant de l'énergie hyperfréquence.

6. Ensemble selon l'une quelconque des revendications précédentes, caractérisé par un moyen placé entre l'élément coupant ( 17 ) et la portion d'extrémité distale de l'élément tubulaire ( 13 ) pour diriger l'énergie, délivrée par l'élément coupant ( 17 ), depuis la portion d'extrémité distale en direction du conduit.

7. Ensemble selon l'une quelconque des revendications précédentes, caractérisé en ce que la partie extérieure ( 69 ) de l'élément coupant ( 17 ) prend des formes progressives en réponse au mouvement progressif du moyen ( 84 ) formant curseur, chacune des formes progressives ayant une configuration particulière.

8. Ensemble selon la revendication 7, caractérisé en ce que la configuration particulière comprend des portions de forme ovale.

9. Ensemble selon la revendication 7, caractérisé en ce que la configuration particulière comprend des portions de forme parabolique.

10. Ensemble selon la revendication 7, caractérisé en ce que la configuration particulière comprend des portions dont la forme est celle d'une partie de coeur.
